# EUROPEAN PATENT APPLICATION

(11) **EP 4 736 888 A1**
(43) Date of publication of application: **06.05.2026**
(21) Application number: 24832535.9
(22) Date of filing: 01.07.2024
(51) Int. Cl.: A61K 48/00, C12N 15/86, C12N 15/113

(54) **COMPOSITION FOR PREVENTING, ALLEVIATING, OR TREATING MITOCHONDRIAL COMPLEX 1 DISORDERS, COMPRISING RECOMBINANT ADENO-ASSOCIATED VIRUS VECTOR LOADED WITH HYDRA NDI1**

(30) Priority: 30.06.2023 KR 20230084924
(71) Applicant: Samsung Life Public Welfare Foundation, Seoul 04348 (KR)
(72) Inventor: KEE, Chang Won, Seoul 06351 (KR); SOHN, Seong Soo, Seoul 06351 (KR); PARK, Kyung Ah, Seoul 06351 (KR)
(74) Representative: Zaccaro, Elisabetta
(86) International application number: PCT/KR2024/009163
(87) International publication number: WO 2025/005759

(57) **Abstract**

The present disclosure relates to a composition for the prevention, alleviation, or treatment of mitochondrial complex 1 disorders, including a recombinant adeno-associated virus (AAV) vector loaded with Hydra NDi1. The inventors have confirmed that the scAAV-Hydra NDi1 of the present disclosure has an effect of restoring cellular respiration reduction caused by rotenone and an effect of inhibiting the apoptosis of LHON patient-derived cells by rotenone. Thus, the scAAV-Hydra NDi1 is expected to be useful for preventing, alleviating, or treating not only LHON but also diseases caused by abnormalities in mitochondrial complex 1.

## Description

### [Technical Field]

The present disclosure relates to a composition for preventing, alleviating or treating mitochondrial complex 1 disorders, including a recombinant adeno-associated virus vector loaded with Hydra NDi1.

### [Background Art]

Mitochondrial genetic diseases caused by mutations in mitochondrial genomes were first reported about 30 years ago, and since then, there have been rapid advances in understanding numerous diseases caused by mutations in the mitochondrial genomes and mitochondrial genetics involved in the transmission of the mitochondrial genomes. Approximately thousands of mitochondrial genomes exist within a cell, and most mutated mitochondrial genomes are mixed with normal genomes within the cell, which is called heteroplasmy. The severity of disease symptoms varies depending on the proportion of mutated genomes, and the higher the proportion of mutated genomes, i.e. the higher the heteroplasmy, the more severe the symptoms. Mitochondrial genome mutations caused in the same manner are highly variable in heteroplasmy according to each tissue of the human body or each subject, and much research is still needed to elucidate mechanisms that control the heteroplasmy.

Leber's hereditary optic neuropathy (LHON) is a mitochondrial disease that causes sudden, painless loss of vision in both eyes due to characteristic damage to the optic nerves at a young age, and was first described in 1871 by the German ophthalmologist, Theodore Leber. The LHON is mainly developed in men (about 80%) more than in women, and in the women, the LHON is developed later and more severely affected. The developed time is usually between the ages of 18 and 30, but the LHON may occur at all ages without warning, and cause simultaneous or sequential loss of vision in both eyes acutely or subacutely. The frequency of occurrence is not yet known exactly. The LHON is the first genetic disease discovered to be inherited due to mutations in mitochondrial DNA (mtDNA), and is a disease that is inherited only through the maternal line. Most patients have a family history from the maternal line, but 40% of the patients have spontaneous cases.

Mitochondria are important intracellular organelles that produce energy (ATP) used for biological activities, and have independent genes, unlike nuclear genes. Most genetic diseases are caused by defects in genes within the nucleus, but some genetic diseases may be caused by abnormalities in mitochondrial genes. A pair of genes in the nucleus is inherited from parents, respectively, but mitochondrial genes are inherited only from the maternal line because mitochondria, which contain paternal genes in the sperm's tail, fall off during fertilization and do not enter the egg. The LHON accounts for 30 to 50% of idiopathic optic neuropathy occurring in both eyes and 2% of legal blindness. Approximately 90% of LHON has one of three point mutations of G11778A (approximately 70% worldwide), G3460A, and T14484C (most common in people of mixed French and Canadian descent), and several other point mutation genes have been identified. Most people have one mutated gene (homoplasic), but rarely have two or more mutated genes (heteroplasic). Although the exact pathogenesis of the disease is unknown, it is considered that a defective mitochondrial gene is involved in a normal cell division and a cell growth process, which causes problems with energy production in optic nerve cells, resulting in cell destruction.

Although drug therapy administering vitamin C, vitamin B₁₂ or idebenone may have an effect of alleviating damage to retinal ganglion cells in patients with the disease, the effect thereof is minimal, so that the drug therapy is not widely used in clinical practice, and has the limitation of not being able to fundamentally prevent the disease. Therefore, there is currently no suitable therapy, and only genetic therapy may be fundamental treatment.

Accordingly, the present inventors produced a recombinant viral vector containing hydra NDi1 to effectively treat mitochondrial disorders, and experimentally demonstrated that the vector was used to prevent, alleviate, and treat mitochondrial disorders, and then completed the present disclosure.

### [Disclosure]

### [Technical Problem]

An object of the present disclosure is to provide a pharmaceutical composition for preventing or treating mitochondrial disorders, including a recombinant viral vector loaded with a hydra NDi1 gene represented by SEQ ID NO: 1.

Another object of the present disclosure is to provide a method for preventing or treating mitochondrial disorders, including administering to a subject a pharmaceutical composition for preventing or treating mitochondrial disorders including a recombinant viral vector loaded with a hydra NDi1 gene represented by SEQ ID NO: 1.

However, technical objects to be achieved by the present disclosure are not limited to the aforementioned objects, and other objects which are not mentioned can be clearly understood to those skilled in the art from the following description.

### [Technical Solution]

In order to achieve the object, an aspect of the present disclosure provides a pharmaceutical composition for preventing or treating mitochondrial disorders, including a recombinant viral vector loaded with a hydra NDi1 gene represented by SEQ ID NO: 1.

Another aspect of the present disclosure provides a method for preventing or treating mitochondrial disorders, including administering a pharmaceutical composition including the vector of the present disclosure as an active ingredient to a subject in need thereof.

Yet another aspect of the present disclosure provides a use of a pharmaceutical composition including the vector of the present disclosure as an active ingredient for the prevention or treatment of mitochondrial disorders.

Still another aspect of the present disclosure provides a use of the vector of the present disclosure for producing a drug for preventing or treating mitochondrial disorders.

### [Advantageous Effects]

The present inventors have confirmed that the scAAV-Hydra NDi1 of the present disclosure has an effect of restoring cellular respiration reduction caused by rotenone and an effect of inhibiting the apoptosis of LHON patient-derived cells by rotenone. Thus, the scAAV-Hydra NDi1 is expected to be useful for preventing, alleviating, or treating not only LHON but also diseases caused by abnormalities in mitochondrial complex 1.

### [Description of Drawings]

FIG. 1 is a schematic diagram showing production of ssAAV-2/DJ-GFP, ssAAV-2/DJ-IRES-GFP, and scAAV-2/DJ-GFP recombinant adeno-associated viruses.
FIG. 2 is a schematic diagram showing production of ssAAV-2/DJ-yeast NDi1, ssAAV-2/DJ-hydra NDi1, and scAAV-2/DJ-hydra NDi1 recombinant adeno-associated viruses.
FIG. 3A shows cleavage maps of recombinant adeno-associated virus vectors used in experiments for confirming the gene expression intensity and stability of ssAAV and scAAV.
FIG. 3B shows results of confirming green fluorescence expression levels of ssAAV-GFP and scAAV-GFP.
FIG. 4 shows results of measuring the oxygen consumption rate (OCR) after adding rotenone to skin fibroblast cells derived from LHON patients infected with ssAAV-IRES-GFP, ssAAV-yeast NDi1, and scAAV-hydra NDi1.
FIGS. 5A to 5D show results of adding rotenone to skin fibroblast cells derived from LHON patients infected with ssAAV-IRES-GFP, ssAAV-yeast NDi1, ssAAV-hydra NDi1, and scAAV-hydra NDi1, and observing the degree of apoptosis on day 1 to day 4 after rotenone treatment.

### [Best Mode]

The terms used in the present disclosure are used for the purpose of description only, and should not be construed to be limited. A singular expression includes a plural expression unless the context clearly indicates otherwise. In the present disclosure, it should be understood that term "including" or "having" indicates that a feature, a number, a step, an operation, a component, a part or a combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by those skilled in the art. Terms which are defined in generally used dictionaries should be interpreted to have the same meaning as the meaning in the context of the related art, and are not interpreted as ideal or excessively formal meanings unless otherwise clearly defined herein.

Hereinafter, the present disclosure will be described in detail.

The present disclosure provides a pharmaceutical composition for preventing or treating mitochondrial disorders, including a recombinant viral vector loaded with a hydra NDi1 gene represented by SEQ ID NO: 1.

In the present disclosure, "NADH dehydrogenase or NADH-ubiquinone oxidoreductase (NDi1)" is NADH dehydrogenase present in the mitochondria of organisms without a large enzyme complex such as mammalian mitochondrial complex 1, and performs a function of mitochondrial complex 1. The NDi1 transfers electrons from NADH to ubiquinone. In an embodiment of the present disclosure, NDi1 derived from *Hydra vulgaris* was used, and the NDi1 derived from *Hydra vulgaris* has a smaller molecular weight than NDi1 derived from *Saccharomyces cerevisiae*. In an embodiment of the present disclosure, a hydra NDi1 gene represented by the following SEQ ID NO: 1 was used.

SEQ ID NO: 1:

agttttagaa tttataccag cagctagtta taagcgcaaa atgccattct ctttatcatc 60

tgttctgtat cggaaaagac ttgttttatt aggaacagga tggggatgtt atagtgtgtt 120

aaaaaatatt aacaagaagc tttatgatgt gattgttgtt agtccacgaa atcattttct 180

ttttacaccc ttgttaaata gcacaactgt tgggacttta gaatttcgaa gcataattga 240

accgattcga aacactaagt ttcgggatga tcatcatttt caattggctg aagcaattca 300

cttaaatcca catgataaat tagttgtttg taaagcagtc tcatctgata aagaatatac 360

tttatcatac gataaagttg taattggtgt tggagctgtt agtaatactt ttggaattcc 420

tggtgtccca aagtatgcat attttttgaa agaaatagca gatgctagaa aaatacgtaa 480

tcaaatcata tctaattttg agcaaagttt gtttccatat gtaaatgaag aggaaaggtt 540

atcactgttg cattttgtca ttgttggtgg tggtccaact ggtattgaat ttggagctga 600

gttgtatgac tttataacac atgatgttgc aagacttttt ccaggagaga agaatgatgt 660

tcatgtaaca ttagttgaag gagattcaat attaccttca tttgaccagc gattacgtaa 720

atttgctgag agaaaaatta cacaaagaga aaatttccac ttaatcaaag attttgttgt 780

ggaagttggt gaaaactatg tgaagttgaa atcaggaaaa gttcttttaa ctaagcttgt 840

agtgtggtca accggtcttg gtcccagaaa gtttatagaa agtttagacc tacctaaagg 900

taaatcaaaa caactcaagg tagatgatca tcttcgtgta gttggttatg acagtatatt 960

tgcaattggt gattgttcat acatagatgg atctccttta ccaagcacgg ctcaagtggc 1020

tgaaagacaa ggtcgatatg ttgcacagta cttatcctta ttagaaacaa attctaaaac 1080

agacagtaaa ccatttatgt ggtccaatgc tggtatgctt gcatatattg gtggatataa 1140

agctgttgca gatcttccaa caaaagctgg gaagataact ggatttaaaa gttggttaat 1200

atggcgatcg gtttatctta ctcgcttagg gagctggaga aatcgaatgc aagttccatt 1260

tgactgggct aggacttttt tttttggcag ggatatatct aggttttaaa ttacaaactt 1320

tatttaatat ttagtatttt ttgtatctta ttaatttgtt tttaa

In the present disclosure, the "recombinant vector" is a genetic construct containing essential regulatory elements operably linked to express a genetic insert, and may be a plasmid vector, a cosmid vector, a bacteriophage vector, or a viral vector. According to an embodiment of the present disclosure, the recombinant vector may be a viral vector, specifically an adenovirus vector, an adeno-associated virus vector, or a retrovirus vector, and more specifically an adeno-associated virus vector, but is not limited thereto.

In the present disclosure, the recombinant viral vector includes the hydra NDi1 gene represented by SEQ ID NO: 1. It is preferred that the hydra NDi1 gene sequence is present in a suitable expression construct. In the expression construct, it is preferred that the hydra NDi1 gene sequence is operatively linked to a promoter.

As used herein, "operatively linked" means a functional linkage between a nucleic acid expression regulatory sequence (e.g., a promoter, a signal sequence, or an array of transcription factor binding sites) and the other nucleic acid sequence, and thus the regulatory sequence regulates transcription and/or translation of the other nucleic acid sequence.

In the present disclosure, the promoter linked to the hydra NDi1 gene sequence may be any promoter capable of initiating expression of hydra NDi1, but it is preferable to select a promoter capable of operating in an animal cell, more specifically, a mammalian cell, to regulate transcription of the hydra NDi1 gene sequence. The promoter includes a promoter derived from a mammalian virus and a promoter derived from the genome of a mammalian cell, and for example, includes a U6 promoter, a T7 promoter, an H1 promoter, a cytomegalovirus (CMV) promoter, an adenovirus late promoter, a vaccinia virus 7.5K promoter, an SV40 promoter, a tk promoter of HSV, an RSV promoter, an EF1 alpha promoter, a metallothionine promoter, a beta-actin promoter, a promoter of a human IL-2 gene, a promoter of a human IFN gene, a promoter of a human IL-4 gene, a promoter of a human lymphotoxin gene, a promoter of a human GM-CSF gene, an inducible promoter, or a tissue-specific promoter (e.g., an albumin promoter), but is not limited thereto. The vector may be produced using a genetic recombination technique well-known in the art, and site-specific DNA cleavage and linkage may use enzymes and the like which are generally known in the art.

In the present disclosure, the adeno-associated virus (AAV) is a single-stranded DNA virus belonging to the parvovirus. The adeno-associated virus is a defective virus that cannot replicate alone, but may replicate and multiply when infected with a helper virus such as adenovirus, vaccinia, or herpesvirus. The adeno-associated virus is able to infect both dividing and non-dividing cells. In an embodiment of the present disclosure, the adeno-associated virus may be a self-complementary AAV (scAAV), which has high expression stability and expression intensity and low immunogenecity.

In the present disclosure, the recombinant viral vector may inhibit cellular respiration reduction or apoptosis caused by abnormalities in mitochondrial complex 1, but is not limited thereto. The abnormalities in mitochondrial complex 1 may be caused by mutations in ND1, ND4, or ND6 genes, but are not limited thereto.

As used herein, "mitochondrial disorders" are diseases caused by mutations in mitochondrial DNA (mtDNA, mitochondrial gene), and various symptoms are developed due to changes in mitochondrial morphology or biochemical properties in various tissues. The mitochondrial disorders may be caused by abnormalities in mitochondrial complex 1, but are not limited thereto. In addition, the mitochondrial disorders may be caused by mutations in one or more genes selected from the group consisting of NADH dehydrogenase subunit 1 (ND1), NADH dehydrogenase subunit 4 (ND4), and NADH dehydrogenase subunit 6 (ND6), but are not limited thereto. In addition, the mitochondrial disorders may be selected from the group consisting of Leber's hereditary optic neuropathy (LHON), mitochondrial encephalopathy with lactic acidosis and stroke-like episode (MELAS) syndrome, neurogenic muscle weakness, myoclonic epilepsy with ragged red fiber (MERRF) syndrome, mitochondrial encephalomyopathy, Leigh syndrome, Kearns-Sayre syndrome, multiple sclerosis, encephalomyelitis, cranial neuritis, peripheral neuropathy, Friedreich's ataxia, Alpers disease, stroke, migraine, psychosis, depression, seizure, dementia, apoplexy, optic atrophy, optic neuropathy, glaucoma, retinitis pigmentosa, cataract, hyperaldosteronism, hypoparathyroidism, myopathy, muscle atrophy, myoglobinuria, hypotonia, myalgia, reduced exercise tolerance, renal tubulopathy, renal failure, liver failure, liver dysfunction, hepatomegaly, sideroblastic anemia, neutropenia, thrombocytopenia, diarrhea, villous atrophy, polyvomiting, dysphagia, constipation, sensorineural hearing loss, mental retardation, epilepsy, Alzheimer's disease, Parkinson's disease, and Huntington's disease, and preferably Leber's hereditary optic neuropathy (LHON), mitochondrial encephalopathy with lactic acidosis and stroke-like episode (MELAS) syndrome, neurogenic muscle weakness, myoclonic epilepsy with ragged red fiber (MERRF) syndrome, mitochondrial encephalomyopathy, Leigh syndrome, and Kearns-Sayre syndrome, and in an embodiment of the present disclosure, Leber's hereditary optic neuropathy (LHON), but are not limited thereto.

In the present disclosure, the Leber's hereditary optic neuropathy (LHON) may be called Leber's optic atrophy, etc., and refers to a disease in which complex 1 in mitochondria does not function properly due to mitochondrial ND1, ND4, or ND6 mitochondria-DNA (mtDNA) mutations, causing abnormalities in the optic nerve system and resulting in blindness.

The content of the vector of the present disclosure in the composition of the present disclosure is able to be appropriately adjusted depending on the symptoms of a disease, the degree of progression of the symptoms, the condition of a patient, etc., and may be, for example, 0.0001 to 99.9 wt% or 0.001 to 50 wt% based on the total weight of the composition, but is not limited thereto. The content ratio is a value based on a dry amount after removing the solvent.

The pharmaceutical composition according to the present disclosure may further include suitable carriers, excipients, and diluents which are commonly used in the preparation of the pharmaceutical composition. The excipients may be, for example, one or more selected from the group consisting of diluents, binders, disintegrants, lubricants, adsorbents, moisturizers, film-coating materials, and controlled-release additives.

The pharmaceutical composition according to the present disclosure may be formulated and used in the form of external preparations, such as powders, granules, sustained-release granules, enteric-coated granules, liquids, eye drops, elixirs, emulsions, suspensions, alcohols, troches, aromatic water, limonades, tablets, sustained-release tablets, enteric-coated tablets, sublingual tablets, hard capsules, soft capsules, sustained-release capsules, enteric-coated capsules, pills, tinctures, soft extracts, dry extracts, fluid extracts, injections, capsules, irrigants, plasters, lotions, pastes, sprays, inhalants, patches, sterile injectable solutions, or aerosols, according to conventional methods, respectively. The external preparations may have formulations, such as creams, gels, patches, sprays, ointments, plasters, lotions, liniments, pastes, or cataplasmas.

The carriers, the excipients, and the diluents that may be included in the pharmaceutical composition according to the present disclosure may include lactose, dextrose, sucrose, oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

In the case of the formulation, the formulations may be prepared by using diluents or excipients, such as a filler, an extender, a binder, a wetting agent, a disintegrating agent, a surfactant, etc., which are commonly used.

The additives of the tablets, powders, granules, capsules, pills and troches according to the present disclosure may be used with excipients such as corn starch, potato starch, wheat starch, lactose, sucrose, glucose, fructose, D-mannitol, precipitated calcium carbonate, synthetic aluminum silicate, calcium hydrogen phosphate, calcium sulfate, sodium chloride, sodium bicarbonate, refined lanolin, microcrystalline cellulose, dextrin, sodium alginate, methylcellulose, sodium carboxymethyl cellulose, kaolin, urea, colloidal silica gel, hydroxypropyl starch, hydroxypropyl methyl cellulose (HPMC) 1928, HPMC 2208, HPMC 2906, HPMC 2910, propylene glycol, casein, calcium lactate and Primojel; and binders such as gelatin, arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, refined shellac, starch, hydroxypropyl cellulose, hydroxypropyl methylcellulose, polyvinyl alcohol, and polyvinyl pyrrolidone.

In addition, the additives may be used with disintegrants such as hydroxypropyl methylcellulose, corn starch, agar powder, methylcellulose, bentonite, hydroxypropyl starch, sodium carboxymethylcellulose, sodium alginate, carboxymethyl cellulose calcium, calcium citrate, sodium lauryl sulfate, anhydrous silicic acid, 1-hydroxypropyl cellulose, dextran, ion exchange resin, polyvinyl acetate, formaldehyde-treated casein and gelatin, alginic acid, amylose, guar gum, baking soda, polyvinyl pyrrolidone, calcium phosphate, gelled starch, arabic gum, amylopectin, pectin, sodium polyphosphate, ethyl cellulose, white sugar, magnesium aluminum silicate, di-sorbitol solution, and light anhydrous silicic acid; and lubricants, such as calcium stearate, magnesium stearate, stearic acid, hydrogenated vegetable oil, talc, lycopodium powder, kaolin, vaseline, sodium stearate, cocoa butter, sodium salicylate, magnesium salicylate, polyethylene glycol 4000, polyethylene glycol 6000, liquid paraffin, hydrogenated soybean oil (Lubri wax), aluminum stearate, zinc stearate, sodium lauryl sulfate, magnesium oxide, macrogol, synthetic aluminum silicate, anhydrous silicic acid, higher fatty acids, higher alcohols, silicone oil, paraffin oil, polyethylene glycol fatty acid ether, starch, sodium chloride, sodium acetate, sodium oleate, dl-leucine, and light anhydrous silicic acid.

Additives in the liquids according to the present disclosure may be used with water, diluted hydrochloric acid, diluted sulfuric acid, sodium citrate, monostearate sucroses, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamin, polyvinylpyrrolidone, ethylcellulose, sodium carboxymethylcellulose, etc.

The syrups according to the present disclosure may be used with a white sugar solution, other sugars or sweeteners, etc., and may also be used with a flavoring agent, a coloring agent, a preservative, a stabilizer, a suspending agent, an emulsifier, a thickener, etc., as needed.

In the emulsions according to the present disclosure, purified water may be used, and an emulsifier, a preservative, a stabilizer, a fragrance, etc. may be used as needed.

The suspensions according to the present disclosure may be used with suspending agents, such as acacia, tragacanth, methylcellulose, carboxymethylcellulose, sodium carboxymethylcellulose, microcrystalline cellulose, sodium alginate, hydroxypropylmethylcellulose (HPMC), HPMC 1828, HPMC 2906, HPMC 2910, and the like, and may be used with surfactants, preservatives, stabilizers, colorants, and fragrances, as needed.

The injections according to the present disclosure may include: solvents such as distilled water for injection, 0.9% sodium chloride injection, Ringer's injection, dextrose injection, dextrose + sodium chloride injection, PEG, lactated Ringer's injection, ethanol, propylene glycol, nonvolatile oils such as sesame oil, cottonseed oil, peanut oil, soybean oil, and corn oil, ethyl oleate, isopropyl myristate, and benzyl benzoate; solubilizers such as sodium benzoate, sodium salicylate, sodium acetate, urea, urethane, monoethylacetamide, butazolidine, propylene glycol, Tweens, nicotinamide, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃), carbon dioxide, sodium metabisulfite (Na₂S₂O₅), sodium sulfite (Na₂SO₃), nitrogen gas (N₂), and ethylenediaminetetraacetic acid; sulfating agents such as sodium bisulfide 0.1%, sodium formaldehyde sulfoxylate, thiourea, disodium ethylenediaminetetraacetate, and acetone sodium bisulfite; analgesics such as benzyl alcohol, chlorobutanol, procaine hydrochloride, glucose, and calcium gluconate; and suspending agents such as CMC sodium, sodium alginate, Tween 80, and aluminum monostearate.

The suppositories according to the present disclosure may be used with base materials, such as cocoa butter, lanolin, Witepsol, polyethylene glycol, glycerogelatin, methylcellulose, carboxymethylcellulose, a mixture of stearic acid and oleic acid, subanal, cottonseed oil, peanut oil, palm oil, cocoa butter + cholesterol, lecithin, lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydroxocote SP, S-70-XXA, S-70-XX75 (S-70-XX95), Hydrokote 25, Hydrokote 711, Idropostal, Massa estrarium (A, AS, B, C, D, E, I, T), Massa-MF, Masupol, Masupol-15, Neosupostal-N, Paramound-B, Suposiro (OSI, OSIX, A, B, C, D, H, L), suppository base type IV (AB, B, A, BC, BBG, E, BGF, C, D, 299), Supostal (N, Es), Wecovi (W, R, S, M, Fs), and Tezester triglyceride bases (TG-95, MA, 57).

Solid formulations for oral administration include tablets, pills, powders, granules, capsules, etc., and these solid formulations are prepared by mixing the extract with at least one excipient, such as starch, calcium carbonate, sucrose or lactose, gelatin, etc. Further, lubricants such as magnesium stearate and talc may be used in addition to simple excipients.

Liquid formulations for oral administration may correspond to a suspension, an oral liquid, an emulsion, a syrup, and the like, and may include various excipients, for example, a wetting agent, a sweetener, an aromatic agent, a preserving agent, and the like, in addition to water and liquid paraffin which are commonly used as simple diluents. Formulations for parenteral administration include sterile aqueous solutions, non-aqueous solvents, suspensions, emulsions, lyophilized preparations, and suppositories. As the non-aqueous solvents and the suspensions, propylene glycol, polyethylene glycol, vegetable oils such as olive oil, injectable ester such as ethyl oleate, and the like may be used.

The pharmaceutical composition of the present disclosure is administered in a pharmaceutically effective amount. As used herein, the "pharmaceutically effective amount" refers to an amount enough to treat a disease at a reasonable benefit/risk ratio applicable to medical treatment. The effective amount level may be determined according to factors including the type and severity of a disease of a patient, the activity of a drug, the sensitivity to a drug, a time of administration, a route of administration, an excretion rate, a duration of treatment, and simultaneously used drugs, and other factors well-known in the medical field.

The pharmaceutical composition according to the present disclosure may be administered as an individual therapeutic agent or in combination with other therapeutic agents, and may be administered sequentially or simultaneously with conventional therapeutic agents, and may be administered singly or in multiple doses. It is important to administer an amount capable of obtaining a maximum effect with a minimal amount without side effects by considering all the factors, which may be easily determined by those skilled in the art.

The pharmaceutical composition of the present disclosure may be administered to a subject through various routes. All methods of administration may be expected, and for example, the pharmaceutical composition may be administered by oral administration, subcutaneous injection, intraperitoneal administration, intravenous injection, intramuscular injection, intrathecal injection, sublingual administration, buccal administration, rectal insertion, vaginal insertion, ocular administration, otic administration, nasal administration, inhalation, spraying through the mouth or nose, dermal administration, transdermal administration, etc.

The pharmaceutical composition of the present disclosure is determined according to a type of drug as an active ingredient, in addition to many related factors, such as a disease to be treated, an administration route, the age, sex, and body weight of a patient, and the severity of a disease.

As used herein, the "subject" refers to a subject in need of treatment for diseases, and more particularly, refers to mammals such as humans or non-human primates, mice, rats, dogs, cats, horses" and cows.

The term "administration" used herein means providing a predetermined composition of the present disclosure to a subject by any suitable method.

As used herein, "prevention" means all actions of suppressing or delaying the onset of a target disease, "treatment" means all actions of improving or beneficially changing a target disease and its resulting metabolic disorder symptoms by administering the pharmaceutical composition according to the present disclosure, and "alleviation" means all actions of reducing parameters related to a target disease, for example, the degree of symptoms, by administering the composition according to the present disclosure.

Hereinafter, preferred Examples are presented to assist in the understanding of the present disclosure. However, the following Examples are only provided to more easily understand the present disclosure, and the contents of the present disclosure are not limited by the following Examples.

### [Modes]

### [Example]

### Example 1. Production of recombinant adeno-associated virus (AAV)

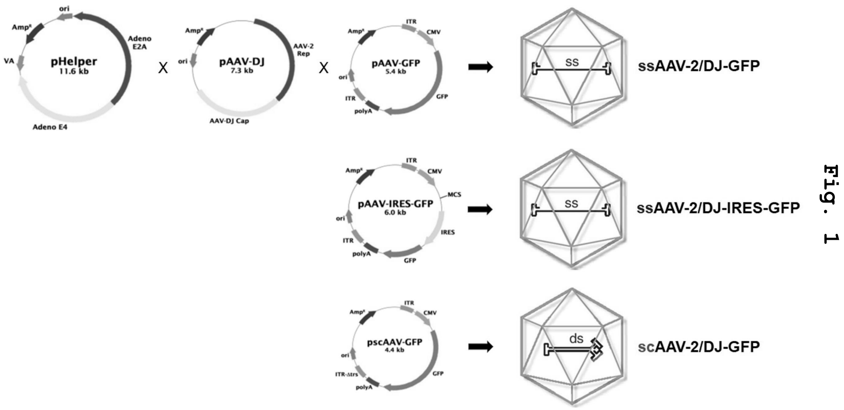

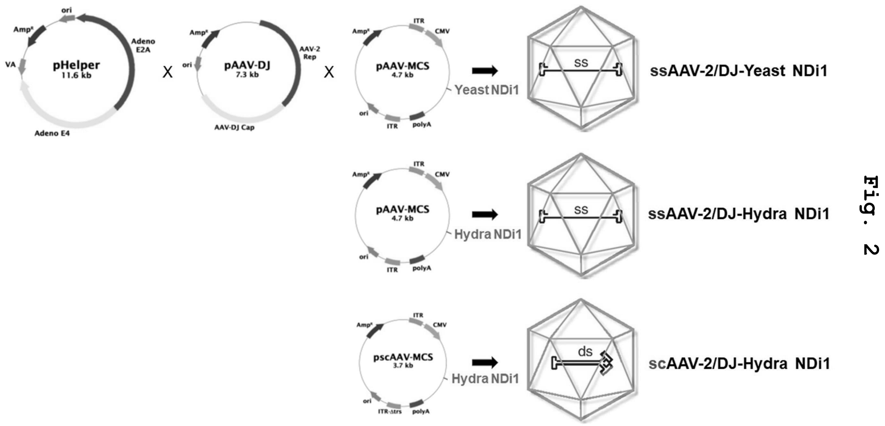

A recombinant adeno-associated virus (AAV) was produced by triple transfection of a vector into HEK293 cells. Specifically, a first vector (pHelper vector) was commonly included, a subtype was determined by a second vector (pRC vector), and single-stranded AAV (ssAAV) and self-complementary AAV (scAAV) were determined by a third vector. In addition, a gene of interest (GOI) was included in the third vector. The pRC vector used pAAV-DJ, and the gene of interest used green fluorescent protein (GFP), internal ribosome entry site (IRES)-GFP, yeast NDi1 (GenBank: X61590.1), and hydra NDi1 (NCBI reference sequence: XM_002156815.3). Individual maps of the vectors used in the experiment were shown in FIGS. 1 and 2.

All vectors for the AAV production were purchased from Cell Biolabs (San Diego, CA), and AAV-293 (Agilent, Santa Clara, CA) was used for 293 cells. The 293 cells were incubated in a 150 mm dish at approximately 90% confluence, and then the first vector, the second vector, and the third vector were mixed well at ratios of 20 µg, 10 µg, and 10 µg, respectively, mixed with a CaCl₂ solution, and then evenly spread on the 293 cells. After 3 days, the 293 cells were scraped with a cell scraper and purified with an AAVpro purification kit (Takara, San Jose, CA), and then the titer was measured with an AAVpro titration kit (Takara, San Jose, CA). Thereafter, the 293 cells were stored at -80°C until use.

### Example 2. Confirmation of gene expression intensity and stability of scAAV

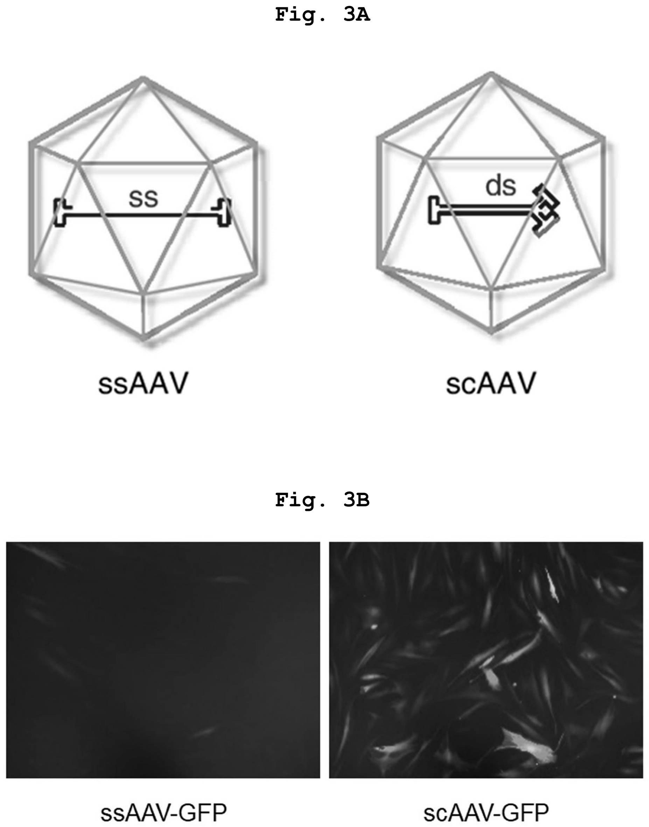

To confirm the intensity and stability of gene expression by scAAV and ssAAV, scAAV-GFP and ssAAV-GFP were infected into fibroblast cells and green fluorescence expression levels were confirmed. At this time, an experiment was conducted using the recombinant AAV shown in FIG. 3A. Specifically, skin fibroblast cells derived from LHON patients were incubated in a 12-well plate at approximately 70% confluence, and then infected by adding approximately 10⁹ viral genomes (vg) of ssAAV-2/DJ-GFP or scAAV-2/DJ-GFP per well, respectively. After 3 days, the expression level of GFP by each recombinant AAV in the infected cells was observed under an FITC filter using an inverted fluorescence microscope. The results were shown in FIG. 3B.

As shown in FIG. 3B, it can be observed that scAAV-GFP expresses green fluorescence more strongly within cells than ssAAV-GFP. This means that scAAV expresses genes stably (stability) and has high gene expression intensity.

### Example 3. Confirmation of cellular respiration restoration effect of scAAV-hydra NDi1

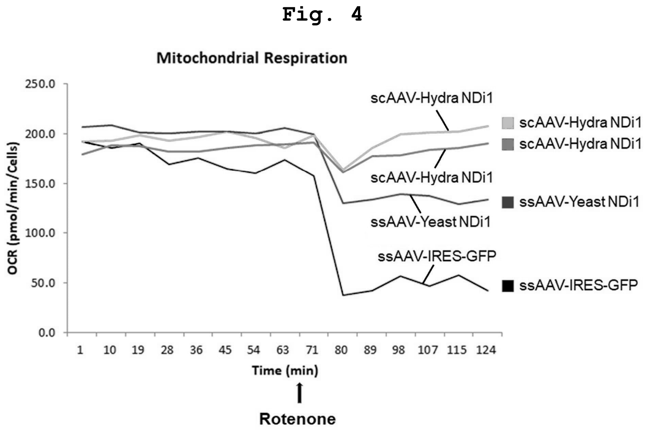

Skin fibroblast cells derived from LHON patients were incubated at approximately 50% confluence in a 24-well plate dedicated for measuring cellular respiration, and then infected with ssAAV-IRES-GFP, ssAAV-yeast NDi1, ssAAV-hydra NDi1, and scAAV-hydra NDi1 at a titer of approximately 10⁸ vg per well, respectively. In the culture medium condition, the oxygen consumption rate (OCR) decreased rapidly by adding rotenone as a complex 1-specific inhibitor. Therefore, after 6 days of AAV infection, rotenone was added to the cells to confirm the inhibitory effect on the cellular respiration reduction by yeast NDi1 and hydra NDi1. The cellular respiration was confirmed by measuring the oxygen consumption rate (OCR) using Seahorse XF-24 from Agilent. The results were shown in FIG. 4.

As shown in FIG. 4, scAAV-hydra NDi1 inhibited the cellular respiration reduction caused by rotenone more efficiently than ssAAV-yeast NDi1.

### Example 4. Confirmation of apoptosis inhibitory effect of scAAV-hydra NDi1

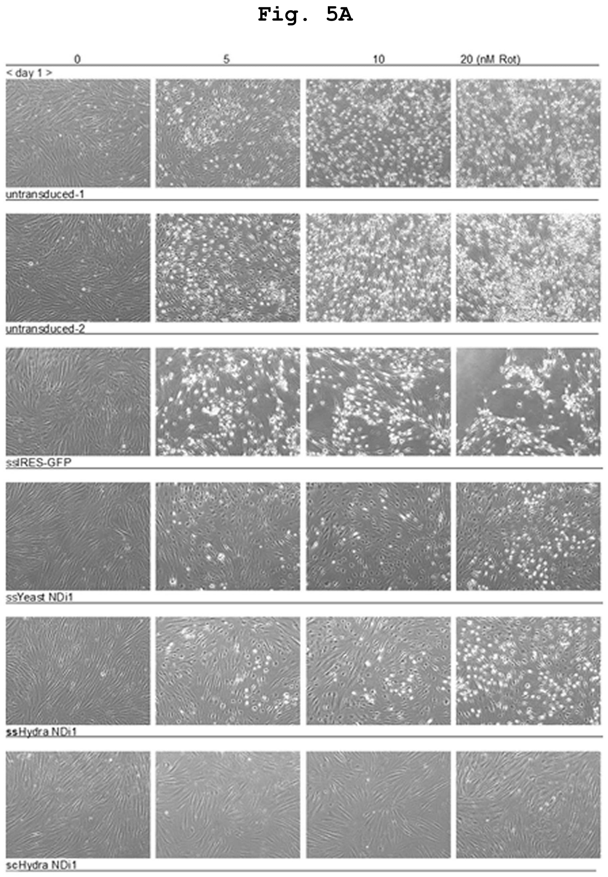

Skin fibroblast cells derived from LHON patients were incubated in a 24-well plate at approximately 70% confluence, and then infected with ssAAV-IRES-GFP, ssAAV-yeast NDi1, ssAAV-hydra NDi1, and scAAV-hydra NDi1 at a titer of approximately 10⁹ vg per well, respectively. After 6 days, rotenone was treated, and apoptosis began to be observed from 1 day after rotenone treatment. The results were shown in FIGS. 5A to 5D. The apoptosis was determined by comprehensively considering the shape and form of individual cells, the degree of attachment to a bottom, the contact intensity with adjacent cells, etc.

As shown in FIG. 5A, after 1 day of rotenone treatment, in an untransduced cell group or ssAAV-IRES-GFP-infected cell group as a control group, some cells began to die at 5 nM rotenone, but cells infected with ssAAV-yeast NDi1 or ssAAV-hydra NDi1 slightly died at 20 nM rotenone. In contrast, under the same conditions, the cell group infected with scAAV-hydra NDi1 hardly died.

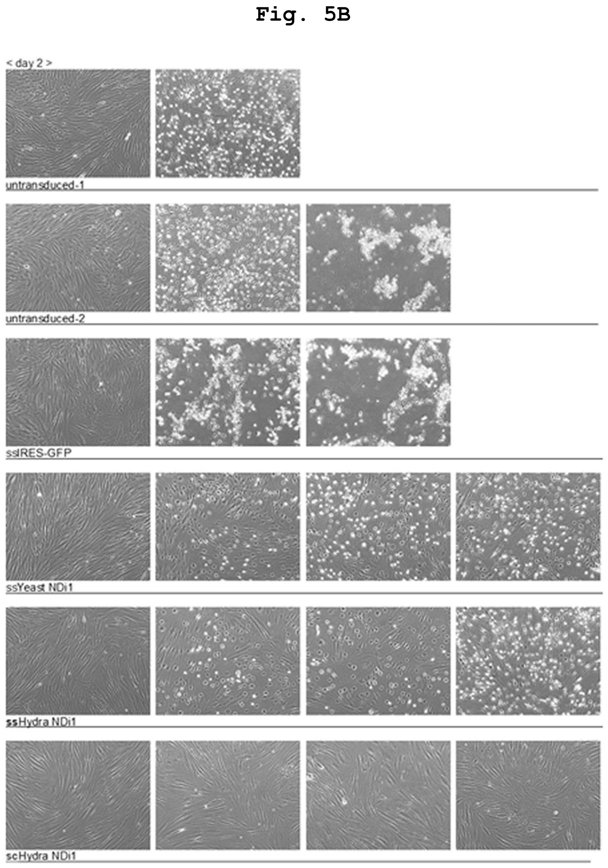

As shown in FIG. 5B, after 2 days of rotenone treatment, in the untransduced cell group or ssAAV-IRES-GFP-infected cell group, almost all cells died even at 5 nM rotenone, but cells infected with ssAAV-yeast NDi1 or ssAAV-hydra NDi1 slightly died at 20 nM rotenone. In contrast, under the same conditions, the cell group infected with scAAV-hydra NDi1 hardly died.

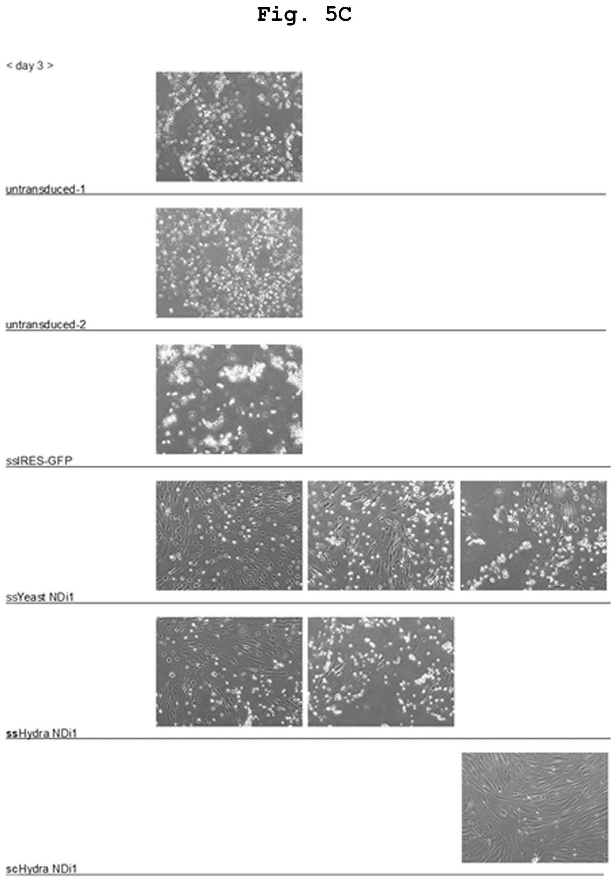

As shown in FIG. 5C, after 3 days of rotenone treatment, in the untransduced cell group or ssAAV-IRES-GFP-infected cell group, cells completely died at 5 nM rotenone, but cells infected with ssAAV-yeast NDi1 or ssAAV-hydra NDi1 began to considerably die even at 10 nM rotenone. In contrast, under the same conditions, the cell group infected with scAAV-hydra NDi1 hardly died even at 20 nM rotenone.

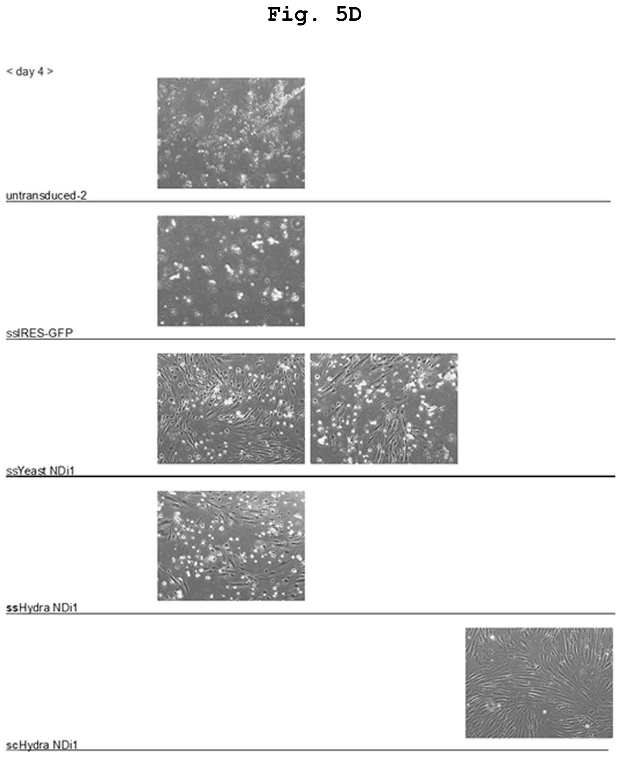

As shown in FIG. 5D, after 4 days of rotenone treatment, cells infected with ssAAV-yeast NDi1 or ssAAV-hydra NDi1 also partially died at 5 nM rotenone and almost died at 10 nM rotenone. In contrast, the cell group infected with scAAV-hydra NDi1 hardly died even at 20 nM rotenone.

This means that scAAV-hydra NDi1 has a significantly excellent inhibitory effect on the apoptosis of LHON patient-derived cells by abnormalities in mitochondrial complex 1 compared to ssAAV-hydra NDi1 and ssAAV-yeast NDi1.

Through the results, it was confirmed that the scAAV-hydra NDi1 of the present disclosure had an effect of restoring cellular respiration reduction caused by rotenone and an effect of inhibiting the apoptosis of LHON patient-derived cells by rotenone. Thus, the scAAV-Hydra NDi1 is expected to be useful for preventing, alleviating, or treating not only LHON but also diseases caused by abnormalities in mitochondrial complex 1.

The aforementioned description of the present disclosure is used for exemplification, and it can be understood to those skilled in the art that the present disclosure can be easily modified in other detailed forms without changing the technical spirit or requisite features of the present disclosure. Therefore, it should be appreciated that the embodiments described above are illustrative in all aspects and are not restricted.

## Claims

1. A pharmaceutical composition for preventing or treating mitochondrial disorder, comprising a recombinant viral vector loaded with a hydra NDi1 gene represented by SEQ ID NO: 1.

2. The pharmaceutical composition for preventing or treating mitochondrial disorder of claim 1, wherein the recombinant virus is an adeno-associated virus.

3. The pharmaceutical composition for preventing or treating mitochondrial disorder of claim 2, wherein the adeno-associated virus is a self-complementary AAV (scAAV).

4. The pharmaceutical composition for preventing or treating mitochondrial disorder of claim 1, wherein the recombinant viral vector inhibits reduction of cellular respiration.

5. The pharmaceutical composition for preventing or treating mitochondrial disorder of claim 1, wherein the recombinant viral vector inhibits apoptosis.

6. The pharmaceutical composition for preventing or treating mitochondrial disorder of claim 1, wherein the mitochondrial disorder is caused by a mutation in one or more genes selected from the group consisting of NADH dehydrogenase subunit 1 (ND1), NADH dehydrogenase subunit 4 (ND4), and NADH dehydrogenase subunit 6 (ND6).

7. The pharmaceutical composition for preventing or treating mitochondrial disorder of claim 1, wherein the mitochondrial disorder is selected from the group consisting of Leber's hereditary optic neuropathy (LHON), mitochondrial encephalopathy with lactic acidosis and stroke-like episode (MELAS) syndrome, neurogenic muscle weakness, myoclonic epilepsy with ragged red fiber (MERRF) syndrome, mitochondrial encephalomyopathy, Leigh syndrome, and Kearns-Sayre syndrome.

8. A method for preventing or treating mitochondrial disorder, comprising administering to a subject a pharmaceutical composition for preventing or treating mitochondrial disorders comprising a recombinant viral vector loaded with a hydra NDi1 gene represented by SEQ ID NO: 1.
